# EUROPEAN PATENT SPECIFICATION

(11) **EP 3 698 730 B1**
(45) Date of publication and mention of the grant of the patent: **26.01.2022**
(21) Application number: 20158897.7
(22) Date of filing: 21.02.2020
(51) Int. Cl.: A61B 17/16

(54) **MILLING TOOL FOR PROSTHETIC SURGERY OPERATIONS**
FRÄSWERKZEUG FÜR PROTHESENCHIRURGIEOPERATIONEN
OUTIL DE FRAISAGE POUR OPÉRATIONS CHIRURGICALES PROTHÉTIQUES

(30) Priority: 22.02.2019 IT 201900002617; 20.08.2019 IT 201900014877
(43) Date of publication of application: 26.08.2020
(73) Proprietor: HPF S.R.L., 33034 Fagagna (IT)
(72) Inventor: Lualdi, Tommaso, 33034 Fagagna (IT)
(74) Representative: Petraz, Gilberto Luigi

(56) References cited:
- EP-A1- 2 915 493
- EP-A1- 3 178 417
- EP-A1- 3 530 216
- WO-A1-2012/136972
- US-A- 5 203 653
- US-A1- 2016 278 792

## Description

### FIELD OF THE INVENTION

The present invention concerns a milling tool for prosthetic surgery operations, able to be used for example to make a bone seating, for example for installing an acetabular hip prosthesis, or a shoulder prosthesis or other, which can be associated with a corresponding handling device.

### BACKGROUND OF THE INVENTION

In general, milling tools are known that can be used during prosthetic surgery operations and are conformed to create coordinated and mating bone seatings suitable for the disposition and implant of corresponding components of surgical prostheses.

In particular, milling tools are known that can be used to make the hemispherical seatings, or in any case those with a spherical cap, suitable for installing coordinated acetabular cups of the hip prostheses.

These known milling tools generally provide an internally hollow milling cap, with sizes correlated to the bone seating to be made and on which a plurality of through apertures are made provided with cutting and protruding edges, in order to perform a mechanical action of excavation on the bone.

In this way, rotating about an axis of rotation, or of milling, and performing a mechanical action of removal of the bone material, this type of milling tools create an impression on the bone of desired size and conformation, substantially corresponding to the milling cap.

It is known that the milling tools as above are operatively associated with handling devices, which can be both of the manual and also the automatic type.

It is also known that these milling tools have to be subjected to washing and sterilizing operations after each surgery, also with the use of washing and sterilizing machines designed for this purpose.

These washing and sterilizing operations naturally entail an increase in the overall costs deriving from the use of these known instruments or tools.

Furthermore, it is possible that these washing and sterilizing operations sometimes prove not to be sufficient, so that contaminating agents, such as viruses, bacteria or suchlike, can in any case remain adherent in particular to the milling cap and, of course, can cause problems for the patient.

These disadvantages related to the effectiveness of the washing and sterilizing operations are also due to a certain structural complexity with which the milling tools are made.

Moreover, the costs of supplying the known milling tools, and also the warehouse stock of these tools, are also usually and undesirably high.

Documents EP-A-2.478.852, US-A-2016/0089158 and EP-A-1.764.046 describe milling instruments for orthopedic surgery, in particular for prosthetic surgery, of a known type. WO 2012/136972 A1 describes a milling tool according to the preamble of claim 1.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore the need to perfect a milling tool for prosthetic surgery operations that can overcome at least one of the disadvantages of the state of the art.

One purpose of the present invention is therefore to provide a milling tool for prosthetic surgery operations which allows to drastically reduce the risk of contamination by external agents, such as viruses, bacteria or suchlike, thus allowing to reduce the risks of contamination for a patient.

It is also a purpose of the present invention to provide a milling tool which allows to limit further washing and sterilizing operations, to be carried out possibly only on determinate zones or parts of the milling tool, thereby optimizing these washing and sterilizing operations, while eliminating them at least for the operative cutting parts of the milling tool.

Another purpose of the present invention is to provide a milling tool for prosthetic surgery operations which is simple and compact in shape, thus allowing production and supply at lower costs compared to what happens with known milling tools.

Another purpose of the invention is to provide a milling tool which is simple, effective and allows to perform the milling operations for which it is intended in an optimal and precise manner.

The Applicant has devised, tested and embodied the present invention to overcome the shortcomings of the state of the art and to obtain these and other purposes and advantages.

### SUMMARY OF THE INVENTION

The present invention is set forth and characterized in the independent claim. The dependent claims describe other characteristics of the invention or variants to the main inventive idea.

These and other aspects, characteristics and advantages of the present disclosure will be better understood with reference to the following description, drawings and attached claims. The drawings, which are integrated and form part of the present description, show some embodiments of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a three-dimensional view of a first embodiment of a milling tool according to an embodiment not forming part of the present invention;
- fig. 2 is a section view of a support part of the milling tool of fig. 1;
- fig. 3 is a three-dimensional and exploded view of a second embodiment of a milling tool according to an embodiment not forming part of the invention;
- fig. 4 is a three-dimensional and assembled view of the second embodiment of the milling tool of fig. 3;
- fig. 5 is a front view of the second embodiment of the milling tool of fig. 4;
- fig. 6 is a section view of the second embodiment of the milling tool considered according to the section line VI-VI of fig. 5;
- fig. 7 is another three-dimensional view of the second embodiment of the milling tool;
- fig. 8 is a three-dimensional and exploded view of a third embodiment of a milling tool according to an embodiment not forming part of the present invention;
- fig. 9 is a longitudinal section and exploded view of the third embodiment of the milling tool;
- fig. 10 is a three-dimensional and assembled view of the third embodiment of the milling tool;
- fig. 11 is a lateral view of a milling tool not forming part of the invention, in accordance with embodiments described here;
- fig. 12 is a section along the line XII-XII of fig. 11;
- fig. 13 is a lateral view with separated parts of a milling tool not forming part of the invention, in accordance with embodiments described here;
- fig. 14 is a section along the line XIII-XIII of fig. 13;
- fig. 15 is a perspective view with separated parts of a milling tool not forming part of the invention, in accordance with embodiments described here;
- fig. 16 is a perspective view with separated parts of a milling tool not forming part of the invention, in accordance with further embodiments described here;
- fig. 17 is a lateral view of a milling tool in accordance with the invention
- fig. 18 is a section along the line XVIII-XVIII of fig. 17;
- fig. 19 is a lateral view with separated parts of a milling tool in accordance with the invention;
- fig. 20 is a section along the line XX-XX of fig. 19;
- figs. 21-22 are perspective views with separated parts of a milling tool in accordance with the invention.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We will now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

Embodiments described using the attached drawings concern a milling tool 20a, 20b, 20c, 20d, 20e, 20f for prosthetic surgery operations, in particular to make concave seatings for the prosthesis implant, for example of the shoulder or hip. The milling tool comprises:
a support part 21, 32 with a substantially hemispherical shape, that is, in the shape of a hemispherical cap, and made of metal, provided, in a single body, with a cutting part 81, 82 having a plurality of protruding cutting elements 22, or blades;
an attachment part 24 made of metal and permanently connected to the support part 21, 32 by welding 31,
an attachment head 29 provided with clamping means 84 able to be selectively activated for a stable and releasable connection to the attachment part 24, the attachment head 29 being able to be connected to a handling device 28.

The attachment part 24 is provided with a central connection seating 27, having a polygonal shape, in particular quadrangular, in order to allow a selectively releasable connection with the attachment head 29.

The polygonal shape, in particular quadrangular, of the central connection seating 27 is advantageous for an effective transmission of the torque required to rotate the milling tool in question.

Advantageously, at least the cutting part is made of titanium.

In possible implementations, the support part 21, 32 is made of titanium.

In other possible implementations, the attachment part 24 is made of titanium.

Advantageously, the milling tool 20a, 20b, 20c, 20d, 20e, 20f can be entirely made of titanium.

Titanium is particularly advantageous since it is hypoallergenic and biocompatible.

Alternatively, one, several or all of the above components can be made of steel.

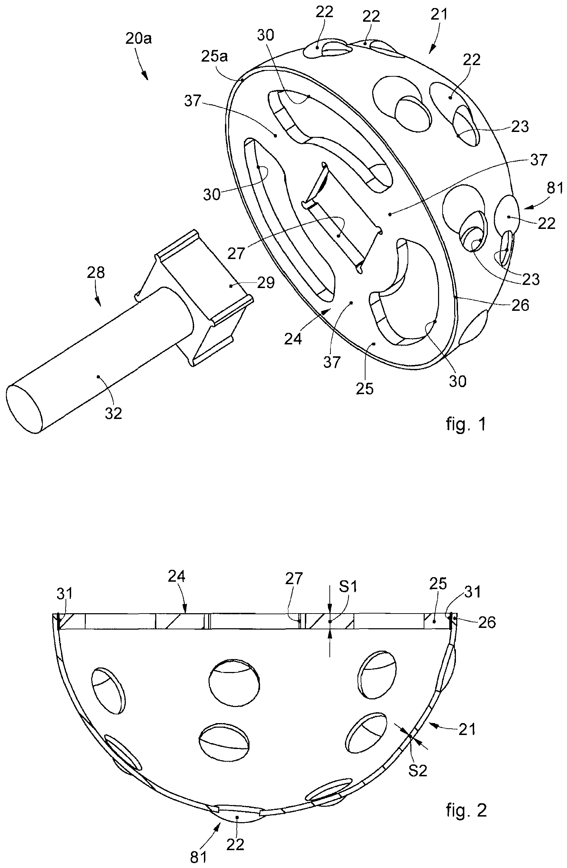

In the embodiments described using figs. 1, 2, 3, 4, 11, 12, 13, 14, 15 and 16, the cutting part 81, 82 and the support part 21, 32 are made in a single body with respect to each other.

In possible implementations, the support part 21 has a thickness S2 comprised between 0.8 mm and 1.2 mm.

In further possible implementations, the attachment part 24 has a thickness S1 comprised between 0.9 mm and 1.1 mm.

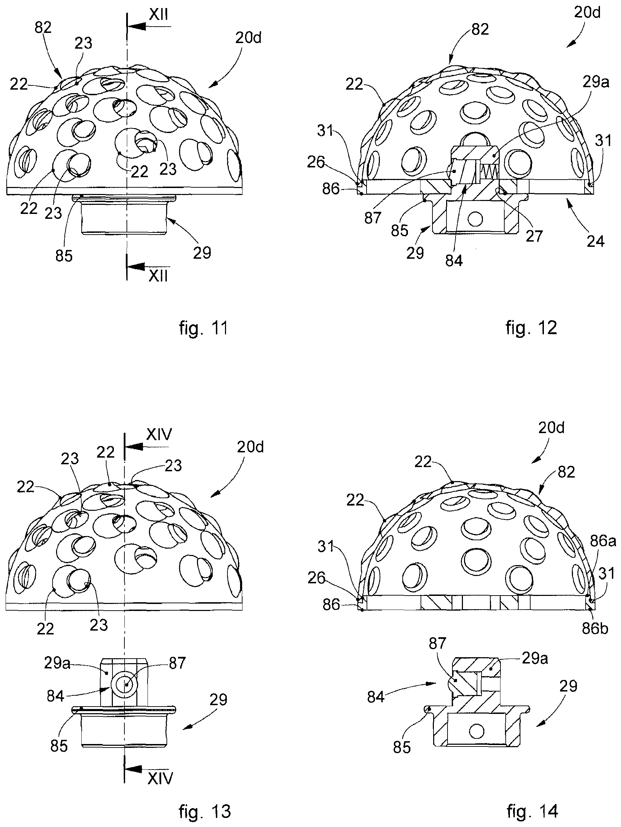

In possible embodiments described using figs. 1, 2, 11, 12, 13, 14, 15 and 16, the attachment head 29 has a shape with a polygonal section, in particular quadrangular, mating with the shape of the connection seating 27 and the clamping means 84 are provided with an elastic retractable element 87 configured to selectively engage the attachment part 24 in correspondence with the connection seating 27 (see for example fig. 12).

The attachment head 29 can be provided for the connection to a handling device 28.

The elastic retractable element 87, therefore, acts as an advantageous selective clamping element with the attachment part 24. This proves to be favorably useful, for example, to prevent undesired detachments or uncoupling between the attachment part 24 and the attachment head 29 associated with the handling device 28.

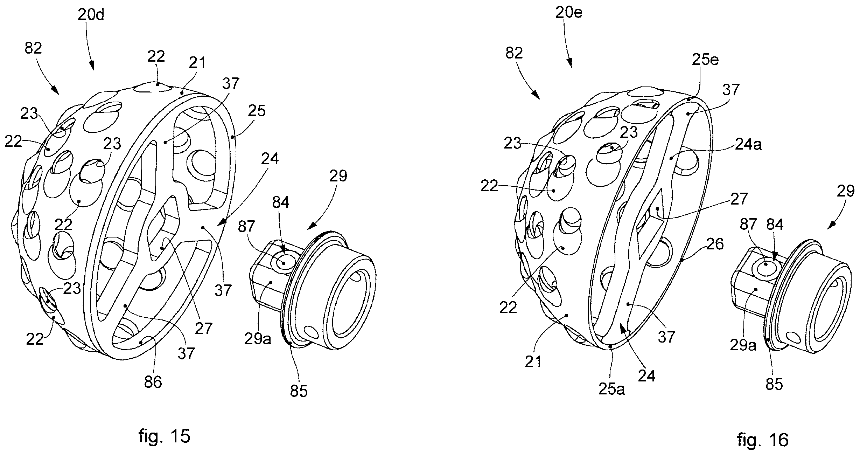

In possible embodiments, the attachment part 24 comprises a peripheral band 25 with a continuous annular shape and three central spokes, or arms, 37 substantially equally spaced apart angularly (see for example figs. 1 and 15).

In other possible embodiments, the attachment part 24 is formed by a bar, or crosspiece, 24a provided with the central connection seating 27 and defining only two diametrically opposite spokes, or arms, 37 (see for example fig. 16).

In possible embodiments described using figs. 11 to 16, the attachment head 29 is provided with a protruding annular bead 85 which abuts against the attachment part 24 (see for example fig. 12). The provision of the protruding annular bead 85 is advantageous since it defines an end-of-travel or safety abutment for the connection between the attachment head 29 and the attachment part 24, also preventing the rise of situations in which, by exerting thrust and pressure on the handling device 28, the attachment head 29 is thrust excessively and in an undesired manner beyond the attachment part 24, toward the inside of the support part 21, with the obvious risks this would entail.

In possible embodiments described using figs. from 11 to 15, the attachment part 24 has a perimeter step edge, or crown, 86 with an annular shape which couples and abuts with an annular peripheral edge 26 of the support part 21, where the attachment part 24 is welded to the support part 21 by means of the welding 31, the perimeter step edge 86 defining an annular portion 86a which narrowd the section of the attachment part 24 recessed inside the support part 21, and an annular abutment portion 86b coupled head-wise with an annular peripheral external edge 26 of the support part 21.

In other possible embodiments described using fig. 16, the attachment part 24 has a peripheral smooth edge 25a which couples with the support part 21 welded and completely recessed, flush with an annular peripheral external edge 26 of the support part 21.

Other embodiments described using figs. 1 to 16 concern milling tools 20a, 20b, 20c, 20d, 20e for prosthetic surgery operations.

The milling tools 20a, 20b, 20c, 20d, 20e comprise:
- a support part 21, 32, 57 with a substantially hemispherical shape,
- an attachment part 24, 33, 68 able to be associated with the support part 21, 32, 57 in order to allow the selectively releasable connection of a handling device 28; and
- a cutting part 81, 82, 83 associated at least with the support part 21, 32, 57 and provided with one or more cutting elements 22.

In accordance with possible embodiments, at least the cutting part 81, 82, 83 can be disposable, and therefore of the type that can be replaced after each use of the milling tool 20a, 20b and 20c. According to one possible solution, at least the cutting part 81, 82, 83 is made of titanium.

The use of this material, also given its relatively low cost, makes it possible to make at least the cutting part 81, 82, 83 disposable. In addition, the use of titanium ensures the high biocompatibility of this material with the human body, preventing problems of postoperative rejection.

In other embodiments, at least the cutting part 81, 82, 83 can instead be reused several times, and therefore is not disposable.

The cutting part 81, 82 and 83 can comprise a plurality of cutting elements 22, or blades, provided to perform the action of removing the material.

In accordance with another embodiment, the support part 21, 32, 57 can have the shape of a hemispherical cap, on which hemispherical cap there is associated the cutting part 81, 82, 83.

The attachment part 24 comprises at least one central connection seating 27 to allow the selectively releasable connection of the handling device 28.

The handling device 28 can be provided with an attachment head 29 with a shape mating with the central connection seating 27, in particular for coupling the attachment head 29, and with a grip 34.

According to variants, the handling device 28 can be provided with coupling portions for coupling into the corresponding coupling elements provided in the attachment part 24.

With reference to the attached drawings, possible embodiments of milling tools are shown, indicated respectively with reference numbers 20a, 20b, 20c, 20d, 20e.

In particular, in one embodiment (figs. 1, 2 and 11-16) the milling tool 20a, 20d, 20e for prosthetic surgery operations provides that the support part 21, the cutting part 81, and the support part 32 are connected to each other in an irremovable manner in order to define as a whole a completely disposable single body.

In accordance with one solution (figs. 1, 2 and 11-16), the cutting part 81 and the support part 21 can be made in a single body with respect to each other.

According to another solution (figs. 1, 2, 11-16), the attachment part 24 and the support part 21 with corresponding cutting part 81 are made in one piece, that is, in a single body, or they can be permanently joined together, for example by welding, so as to constitute a single disposable assembly.

In accordance with possible solutions (figs. 1, 2, 11-16), the attachment part 24, the support part 21 and the cutting part 81 can all be made of titanium, making the whole milling tool 20a, 20d, 20e completely disposable and biocompatible with surgical operations. In possible solutions (figs. 1, 2 and 11-16), the support part 21 has a substantially hemispherical shape and is internally hollow. The cutting elements 22, preferably, can be uniformly distributed on the surface of the support part 21 and through apertures 23 are made in correspondence with them.

The cutting elements 22 can have various shapes and have the function of cutting or milling the bone component, while the through apertures 23 allow the removal of bone residues from the cutting or milling zone.

According to a possible solution, the cutting elements 22 defined above are made in the thickness of the support part 21 and are each provided with at least one cutting edge in order to allow the removal of the material.

The support part 21 is provided with an annular end edge 26 that has a circular shape in which the attachment part 24 is connected, in an irremovable manner.

In accordance with one solution (figs. 1, 2 and 11-16), the attachment part 24 has a discoid shape and is provided with an annular, or peripheral, band 25 with a shape and size mating with the annular edge 26 of the support part 21. In particular, it can be provided that the annular band 25 of the attachment part 24 is in contact and integrally attached to the annular edge 26 of the support part.

In particular, the annular band 25 of the attachment part 24 can be positioned in part of the hemispherical cavity defined by the support part 21, that is, it can be at least partly recessed in it. This positioning of the attachment part 24 allows to obtain an increase in the containing rigidity of the support part 21 preventing it from collapsing in correspondence with the annular edge 26. The attachment part 24 can also comprise spokes, or arms, 37 (see figs 1, 2 and 11-16) between which through apertures 30 are made which have a lightening function and allow the removal of the bone residues from the cutting or milling zone.

In accordance with some solutions (figs. 1, 2 and 11-16), the attachment part 24 has a thickness S1 comprised between 0.9 mm and 1.1 mm, and preferably of about 1 mm, see fig. 2. These thicknesses are, in fact, sufficient to allow the transmission of the torque exerted by the handling device 28, preventing damage to the attachment part 24 and also making this part disposable, reducing the waste of material.

The support part 21, in accordance with possible embodiments with reference for example to figs. 1 and 2, which as stated can have the shape of a hemispherical cap, can have a thickness S2 comprised between 0.3 mm and 0.8 mm and preferably about 0.5 mm.

It has been found that these thickness values S2 of the support part 21 guarantee a perfect circularity of the annular edge 26 of the support part 21, even while the milling tool 20a is being used.

In other embodiments, as stated, the thickness S2 of the support part 21 can be comprised between 0.8 mm and 1.2 mm and preferably be about 1 mm, particularly when the support part 21 and the corresponding cutting part 81, 82 can be reused several times as described above, that is, they are not disposable.

The annular band 25 of the attachment part 24 and the annular edge 26 of the support part 21 can be joined, as mentioned, by welding 31.

The milling tool 20a, 20d, 20e therefore has a support part 21 with a cutting part 81 and an attachment part 24 which can be reused several times, that is, not disposable, or can be completely disposable, that is, they are replaced after each use, depending on requirements.

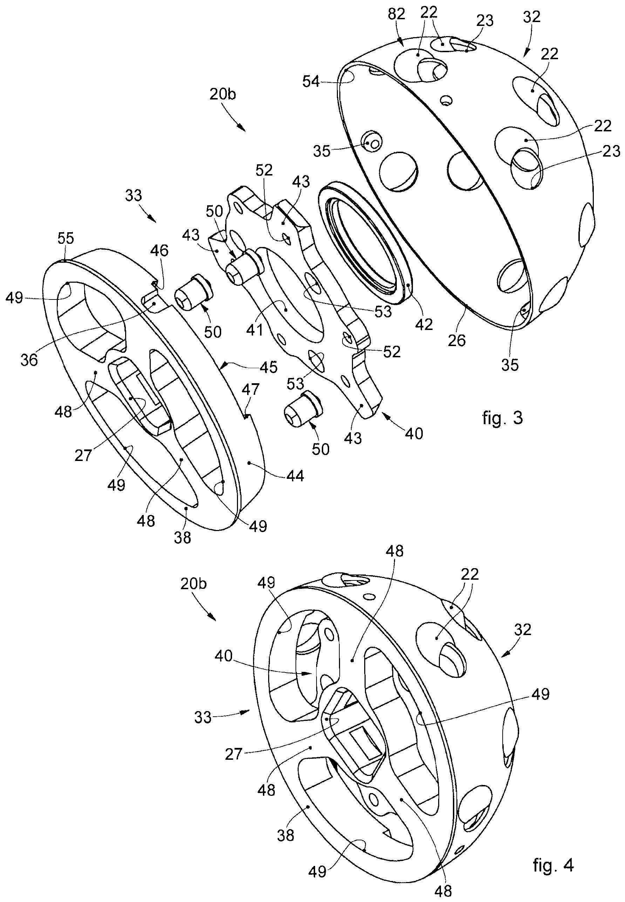

In another embodiment (figs. 3-7) the milling tool 20b comprises the support part 32 provided with the cutting part 82 and the attachment part 33.

Also in this case, by way of example, the cutting part 82 and the support part 32 can be made in a single body with respect to each other.

In particular, it can be provided that the cutting part 82 is formed by the cutting elements 22, variously positioned and made on the support part 32.

In this case, the support part 32 and the cutting part 82 are disposable, while the attachment part 33 can be reusable and therefore can be selectively connected to the support part 32 in a removable manner.

The support part 32 has the shape of a hemispherical cap and therefore comprises the annular edge 26 that defines a circular aperture 54 into which the attachment part 33 is inserted.

The support part 32 and the attachment part 33 are provided with connection means, selectively releasable, in order to allow the selective connection and the subsequent separation, for example after use, of the support part 32 with respect to the attachment part 33.

The support part 32 internally comprises, that is, in the concavity defined by the hemispherical cap, a plurality of teeth 35 protruding radially and configured to engage with corresponding seatings 36 made in a disk 38 of the attachment part 33.

The disc 38 comprises a protruding annular edge 55 on which the annular edge 26 of the support part 32 rests in abutment.

The teeth 35 are preferably made in the proximity of the annular edge 26 of the support part 32.

The teeth 35 can be made by plastic deformation of the support part 32 in order to generate a protrusion toward the inside of the hemispherical cap.

For example, it is possible to provide three teeth 35, disposed equally distanced one from the other in the proximity of the annular edge 26 of the support part 32.

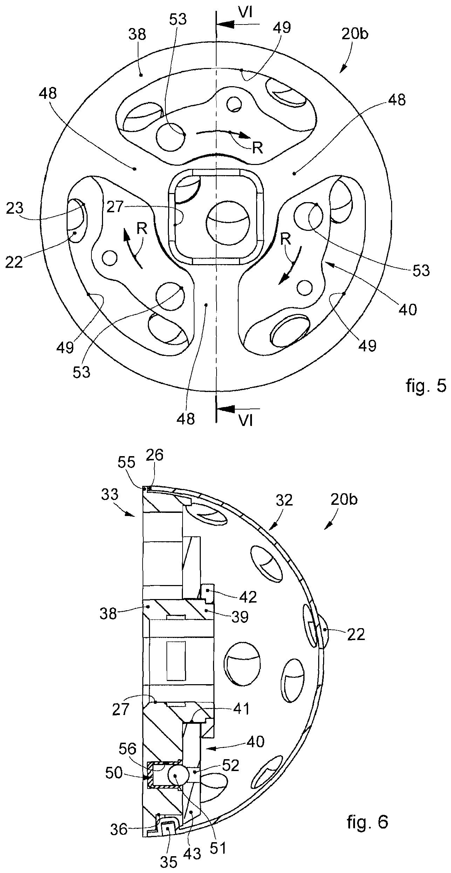

The disc 38, see in particular fig. 6, comprises a cylindrical support 39 located, during use, in the cavity of the spherical cap of the support part 32.

On the cylindrical support 39 there is positioned a rotatable clamping element 40, provided for this purpose with a through hole 41 in which the cylindrical support 39 is inserted.

This cylindrical support 39 can be threaded at the protruding end, so as to engage with a closing ring 42, which is internally threaded.

Once the clamping element 40 has been inserted on the cylindrical support 39, the ring 42 is screwed to the cylindrical support 39, so as to keep the clamping element 40 in the correct position and allow it to rotate with respect to the cylindrical support 39 and therefore to the disk 38.

The clamping element 40 comprises a plurality of spokes 43 protruding radially, and having an extension such as to reach, in a clamping position, in correspondence with the seatings 36 made on the disc 38.

Each of the spokes 43 has the function of preventing the attachment part 33 from separating from the support part 32, once the seatings 36 of the disc 38 have been correctly positioned on the corresponding teeth 35, as shown in fig. 6.

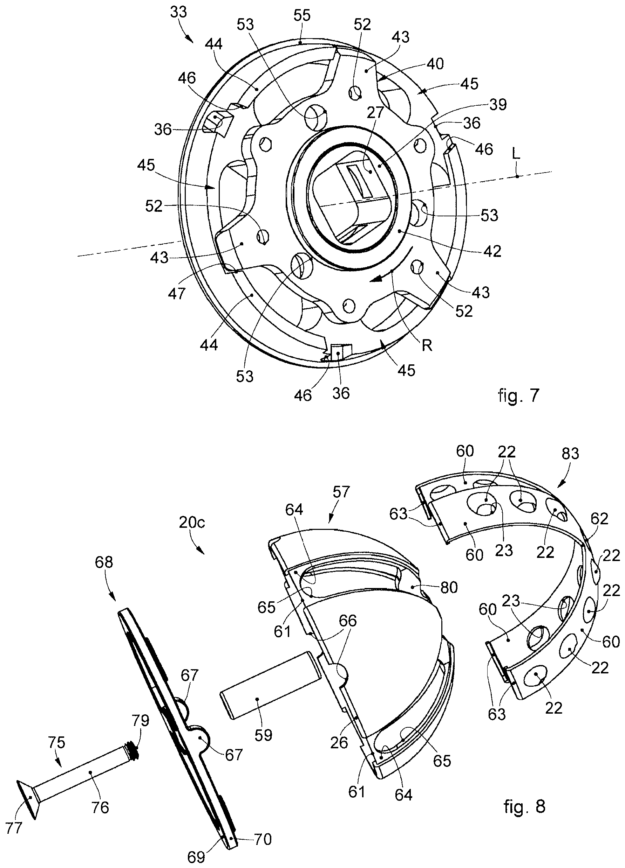

With particular reference to fig. 7, it can be observed that on the lateral wall 44 of the disc 38 arch-shaped grooves 45 are made, along which the spokes 43, integral with the clamping element 40, can rotate.

The clamping element 40 is therefore substantially rotatable with respect to an axis L, in order to make a rotation in the direction R, in one sense or the other, of an amplitude defined by the extension of the arch-shaped grooves 45.

Each of the arch-shaped grooves 45 comprises two end-of-travel ends 46 and 47 which define two limit positions of the spokes 43 of the clamping element 40: one active position in which each spoke 43 is located in correspondence with the seating 36 of the disc 38, and one inactive position, in which the clamping element 40 is inoperative and allows reciprocal separation of the attachment part 33 with respect to the support part 32.

The disc 38 also comprises spokes 48 between which through apertures 49 are made.

The through apertures 49 allow to interact from the outside with the clamping element 40, in order to be able to rotate it at least into the active position and at least into the inactive position.

In the spokes 48, see the section of fig. 6, there are made seatings 56 for housing a series of pins 50 for positioning the clamping element 40.

The pins 50 are provided with a spherical or hemispherical head 51 suitable to engage, for example in a snap-in manner, in corresponding holes 52 made on the spokes 43 of the clamping element 40, in particular in the position in which the spokes 43 are located in correspondence with the seatings 36 of the disc 38, therefore in the active clamping position.

The pins 50 allow to firmly keep the clamping element 40 in position, at least when it is in the active clamping position.

Further holes 53 could also be made on the clamping element 40, suitable to allow its engagement with a rotation tool or device, or suchlike.

Looking for example at fig. 5, it can be assumed that, from the outside of the milling tool 20b, a suitable tool can be inserted through the through apertures 49 and engage in the holes 53 to allow the rotation R of the clamping element 40.

Substantially, therefore, the disc 38 of the attachment part 33 can already be supplied with the clamping element 40 mounted and rotatable in one sense or the other with respect to the disc 38, as a function of the extension of the arch-shaped grooves 45. The disc 38 and the clamping element 40 are kept adjacent and in position thanks to the ring 42.

The attachment part 33 with the disc 38, clamping element 40 and ring 42 appears, for example, as in fig. 7, so that the seatings 36 are not covered by the spokes 43 of the clamping element 40.

The attachment part 33 is then inserted into the support part 32 through the aperture 54, and the seatings 36 of the disk 38 are positioned on the teeth 35.

The insertion of the attachment part 33 is completed when the annular edge 55 arrives in abutment on the annular edge 26 of the support part 32 and the seatings 36 are completely positioned on the teeth 35.

Once the positioning is completed, as for example in fig. 4, the clamping element 40 is rotated in the direction R, fig. 7, so that the spokes 43 move in correspondence with the seatings 36, so that the teeth 35 will be positioned between the seatings 36 and the spokes 43, see for example the section of fig. 6: the clamping element 40, by means of the spokes 43, is in abutment on the teeth 35, preventing the extraction of the attachment part 33.

Therefore, the clamping element 40, in this active or clamping position, interferes with the teeth 35, in particular by means of the spokes 43.

The attachment part 33, in this position, is firmly connected to the support part 32 and cannot therefore be disconnected from it. The correct positioning of the attachment part 33 on the support part 32 is also guaranteed by the engagement of the holes 52 of the spokes 43 on the pins 50.

The disc 38 provided with a rotatable clamping element 40 therefore represent a non-limiting example of possible means for the releasable connection of the attachment part 33 to the support part 32.

In order to separate the attachment part 33 from the support part 32, the clamping element 40 will be rotated in the opposite sense to the clamping rotation R, so that the spokes 43 are no longer abutting on the tooth 35 of the support part 32.

In this embodiment of the milling tool 20b, therefore, the support part 32 and the cutting part 82 are disposable, while the attachment part 33, comprising the disc 38 and the clamping element 40, is reusable.

Fig. 8, fig. 9 and fig. 10 show another embodiment of the milling tool 20c.

The milling tool 20c comprises the support part 57 in the form of a hemispherical cap configured to house the cutting part 83.

Protruding cutting elements, such as for example the cutting elements 22 of fig. 1 or fig. 3, can be positioned, or made on the cutting part 83, in the proximity of the through apertures 23.

In this embodiment of the milling tool 20c, only the cutting part 83 is disposable, while the other parts of the milling tool 20c are reusable.

The cutting part 83 is formed by a plurality of branches 60, with an arched shape, configured to be positioned in corresponding arch-shaped seatings 64 made on the hemispherical surface of the support part 57.

The branches 60 are joined at a common end 62 and are provided, at their free ends, with an edge 63, folded toward the inside of the milling tool 20c, in particular in the radial direction.

It is possible to provide that the cutting part 83 is formed for example by four branches 60, reciprocally equidistant from each other.

The seatings 64, configured to accommodate the branches 60 to size, can preferably be provided with through apertures 65, located during use in correspondence with the internal side of the cutting elements 22. The through apertures 65 allow to discharge the material which is removed by the cutting elements 22 into the concavity of the spherical cap.

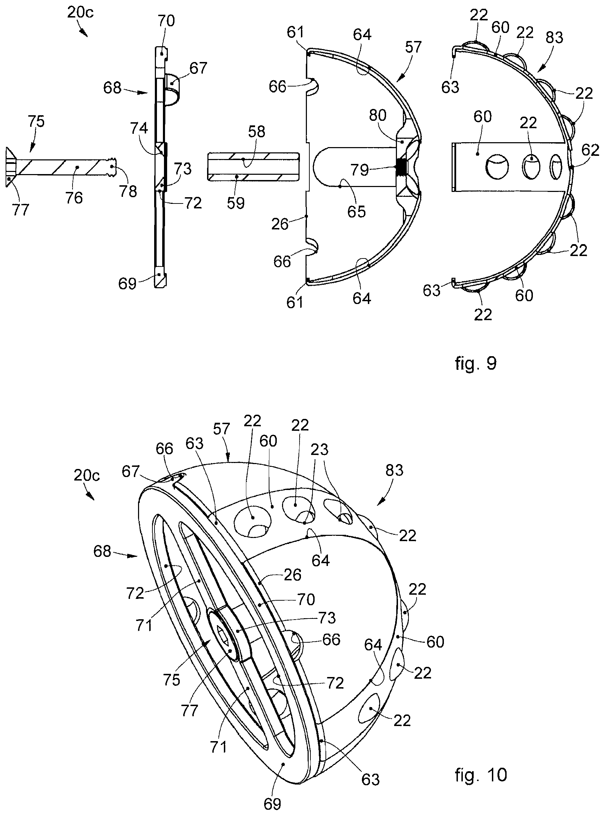

Each of these seatings 64 comprises an edge 61, inside which the edge 63 of the corresponding branch 60 is positioned, so that the cutting part 83 is correctly positioned on the support part 57, as for example in fig. 10.

For this purpose, in the positioning of the cutting part 83 on the support part 57, the branches 60 widen slightly and elastically, until the edges 63 are correctly positioned under the edges 61 of the seatings 64.

The support part 57 is also provided, on the annular edge 26, with one or more notches 66, for example with an arched shape, configured to engage with elements 67 protruding from an attachment part 68.

The attachment part 68 comprises a disk 69, on the annular edge 70 of which the protruding elements 67 are made.

The protruding elements 67 are inserted in the notches 66 of the support part 57, so as to guarantee the correct positioning of the attachment part 68 in the support part 57, and in order to prevent their reciprocal rotation.

The disc 69 of the attachment part 68 comprises spokes 71 between which through apertures 72 are made, joined in a central part 73.

The central part 73 is provided with a through hole 74, suitable to allow the passage of the stem 76 of a pin 75 and to house the head 77 to size.

The head 77 can have a truncated cone shape, and the through hole 74 can consequently be provided with a corresponding truncated cone shape.

The stem 76 of the pin 75 comprises, at the opposite end with respect to that of the head 77, a threaded part 78, configured to be screwed with a corresponding threaded part 79 made inside the support part 57, in particular in a top portion 80 of the support part 57.

The attachment part 68 also comprises a spacer element 59 provided with a through hole 58 and suitable to rest on one side on the central part 73 of the disk 69 and, on the other side, on the top portion 80 of the support part 57.

When assembling the milling tool 20c, firstly, preferably, the cutting part 83 is positioned on the support part 57, therefore with the branches 60 correctly positioned in the seatings 64.

The attachment part 68 is then assembled and connected to the support part 57.

The stem 76 of the pin 75 is inserted in the through hole 74 of the disk 69 and then into the through hole 58 of the spacer element 59, so that the threaded part 78 exits from the spacer element 59 and can engage with the threaded part 79 of the top portion 80 of the support part 57.

The protruding elements 67 of the disc 69 are naturally inserted into the corresponding notches 66.

Furthermore, the annular edge 70 of the disc 69 abuts at least against the annular edge 26 of the support part 57.

The milling tool 20c will then appear, in its assembled and operational configuration, as in fig. 10.

The disk 69 that can be associated with the pin 75 which can be screwed into the support part 57 therefore represents a non-limiting example of possible means for the releasable connection of the attachment part 68 to the support part 57.

To the disk 69 of the attachment part 68 there can be connected a corresponding handling device, provided with suitable elements for releasable connection, for example, to the spokes 71 of the disk 69.

The milling tools 20a, 20b, 20c, 20d, 20e can therefore have at least the cutting parts 81, 82, 83 of the disposable type, or which can be reused, therefore not disposable.

In the case of the milling tool 20a, 20d, 20e the support part 21 and the attachment part 24 can also be disposable, or reusable, therefore not disposable. In the case of the milling tool 20b, in addition to the cutting part 82, the support part 32 can also be disposable. In the case of the milling tool 20c, only the cutting part 83 can be disposable.

The present milling tool 20a, 20b, 20c, 20d, 20e, or at least the support parts 21, 32, 57 and the cutting parts 81, 82, 83, are preferably made of titanium so as to be biocompatible with the body and prevent rejection problems for the patient.

In particular, the titanium used can be grade "0" or grade "1".

Furthermore, titanium guarantees high mechanical resistance in relation to weight, which can be advantageously contained.

It is clear that modifications and/or additions of parts may be made to the milling tool 20a, 20b, 20c, 20d, 20e for prosthetic surgery operations as described heretofore

It is also clear that, although the above has been described with reference to some specific examples, a person of skill in the art shall certainly be able to achieve many other equivalent forms of milling tool 20a, 20b, 20c, 20d, 20e for prosthetic surgery operations, having the characteristics as set forth in the claims and hence all coming within the field of protection defined thereby.

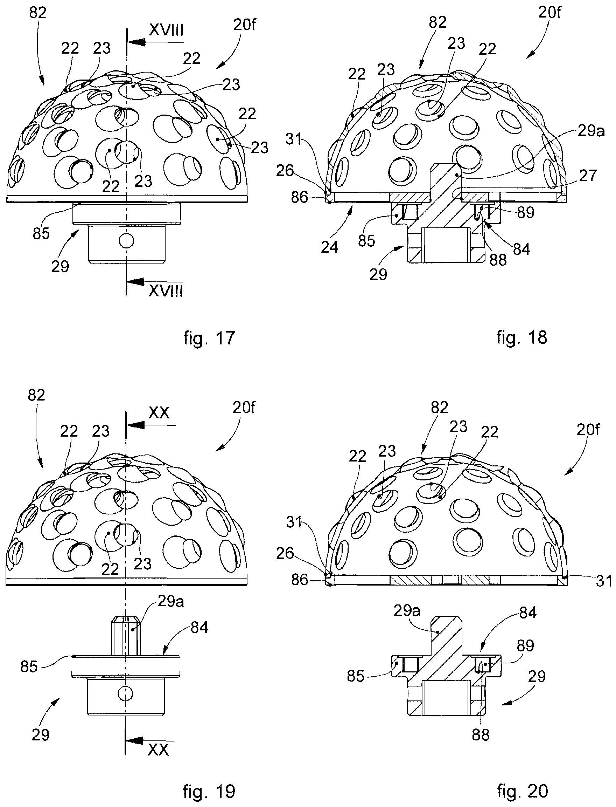

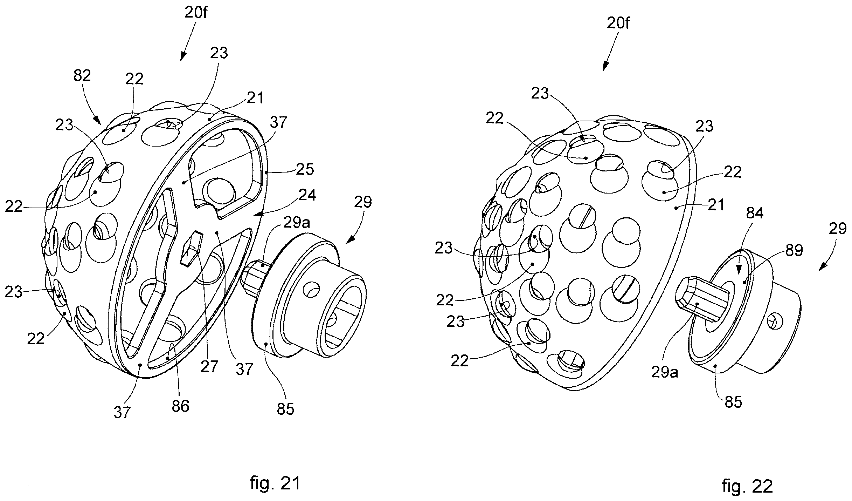

Embodiments shown in figs. 17-22 concern a milling tool 20f for prosthetic surgery operations, in particular to make concave seatings for prosthesis implants, for example of the shoulder or hip.

The milling tool 20f has characteristics that can be combined with any of the characteristics whatsoever described with reference to the milling tools 20a, 20b, 20c, 20d, 20e with the exception that the attachment part 24 of the milling tool 20f is provided with a central connection seating 27, with a hexagonal shape in order to allow a selectively releasable connection with a respective attachment head 29 with a hexagonal-shaped section mating with the shape of the connection seating 27.

In particular, the attachment head 29 is provided with a connection portion 29a projecting from the protruding annular bead 85 and having the hexagonal-shaped section as above.

The hexagonal shape of the central connection seating 27 and of the attachment head 29 allows to distribute the connection force between the attachment head 29 and the support part 21 in a more homogeneous manner. With the same transmitted force, the hexagonal shape allows to reduce the sizes of the connection seating 27.

In accordance with some embodiments, the clamping means 84 of the attachment head 29 comprise a magnetic element 89 configured to selectively abut with the attachment part 24 in correspondence with the connection seating 27.

In accordance with some embodiments, shown in fig. 18 and fig. 20, the attachment head 29 is provided with a throat 88, made on the protruding annular bead 85 and circumscribed to the connection portion 29a, in which the magnetic element 89 is positioned.

The magnetic element 89 has an annular shape substantially mating with the shape of the throat 88.

Advantageously, the presence of the magnetic element 89 allows to transmit a uniform connection force on the attachment part 24, and not localized precisely as in the case of the elastic retractable element 87. In fact, the annular shape of the magnetic element 89 allows to act on the entire perimeter zone around the connection seating 27.

Although the magnetic element 89 is only described with reference to the milling tool 20f, it can be adapted for any milling tool 20a, 20b, 20c, 20d, 20e as previously described.

In the following claims, the sole purpose of the references in brackets is to facilitate reading: they must not be considered as restrictive factors with regard to the field of protection claimed in the specific claims.

## Claims

1. Milling tool for prosthetic surgery operations, comprising:
a support part (21, 32) with a substantially hemispherical shape and made of metal, provided with a cutting part (81, 82) having a plurality of protruding cutting elements (22);
an attachment part (24) made of metal and permanently connected to said support part (21, 32) by welding (31),
an attachment head (29) provided with clamping means (84) being able to be selectively activated for a stable and releasable connection to said attachment part (24), said attachment head (29) being able to be connected to a handling device (28),
said attachment part (24) being provided with a central connection seating (27),
in order to allow a selectively releasable connection with said attachment head (29),
wherein the attachment head (29) comprises:
a protruding annular bead (85) which abuts against the attachment part (24),
a connection portion (29a) projecting from the protruding annular bead (85)
**characterised in that**
the central connection seating (27) has a hexagonal shape and the connection portion (29a) has a hexagonal-shaped section,
the clamping means (84) comprises a magnetic element (89) configured to selectively go into magnetic contact with the attachment part (24), in correspondence with the connection seating (27),
wherein the attachment head (29) comprises
a throat (88), made on the protruding annular bead (85) and circumscribed to the connection portion (29a),
and wherein the magnetic element (89) is positioned in the throat (88).

2. Milling tool as in claim 1, **characterized in that** at least said cutting part (81, 82) is made of titanium.

3. Milling tool as in claim 1 or 2, **characterized in that** the support part (21, 32) is made of titanium.

4. Milling tool as in claim 1, 2 or 3, **characterized in that** the attachment part (24) is made of titanium.

5. Milling tool as in any claim hereinbefore, **characterized in that** it is entirely made of titanium.

6. Milling tool as in any claim hereinbefore, **characterized in that** said cutting part (81, 82) and said support part (21, 32) are made in a single body with each other.

7. Milling tool as in any claim hereinbefore, **characterized in that** said support part (21) has a thickness (S2) comprised between 0.8 mm and 1.2 mm.

8. Milling tool as in any claim hereinbefore, **characterized in that** said attachment part (24) has a thickness (S1) comprised between 0.9 mm and 1.1 mm.

9. Milling tool as in any claim hereinbefore, **characterized in that** said attachment head (29) has a hexagonal section shape, mating with the shape of said connection seating (27).

10. Milling tool as in any claim hereinbefore, **characterized in that** said attachment part (24) comprises a peripheral band (25) of a continuous annular shape and three central spokes (37) substantially equally spaced apart angularly.

11. Milling tool as in any one of the previous claims from 1 to 9, **characterized in that** said attachment part (24) is formed by a bar provided with said central connection seating (27) and defining only two diametrically opposite spokes (37).

12. Milling tool as in any claim hereinbefore, **characterized in that** said attachment part (24) has a perimeter step edge (86) of annular shape which couples and abuts with an annular peripheral edge (26) of said support part (21), where said attachment part (24) is welded to said support part (21), the perimeter step edge (86) defining an annular portion (86a) which narrows the section of said attachment part (24) recessed inside said support part (21), and an annular abutment portion (86b) coupled head-wise with an annular peripheral external edge (26) of said support part (21).

13. Milling tool as in any one of the previous claims from 1 to 11, **characterized in that** said attachment part (24) has a peripheral smooth edge (25a) which couples with said support part (21) welded and completely recessed flush with an annular peripheral external edge (26) of said support part (21).

14. Milling tool as in any claim hereinbefore, **characterized in that** said magnetic element (89) has an annular shape substantially mating with the shape of the throat (88).

15. Milling tool as in claim 14, **characterized in that** the magnetic element (89) is configured to transmit a uniform connection force on the attachment part (24), the annular shape of the magnetic element (89) allowing to act on the entire perimeter zone around the connection seating (27).

## Patentansprüche

1. Fräswerkzeug für prothetisch-chirurgische Operationen, umfassend einen im Wesentlichen halbkugelförmigen Trägerteil (21, 32) aus Metall, der mit einem Schneidteil (81, 82) versehen ist, der eine Vielzahl von vorstehenden Schneidelementen (22) aufweist; einen Befestigungsteil (24) aus Metall, der durch Schweißen (31) dauerhaft mit dem Trägerteil (21, 32) verbunden ist, einen Befestigungskopf (29), der mit Klemmmitteln (84) versehen ist, die selektiv für eine stabile und lösbare Verbindung mit dem Befestigungsteil (24) aktivierbar sind, wobei der Befestigungskopf (29) mit einer Handhabungsvorrichtung (28) verbindbar ist, wobei der genannte Befestigungsteil (24) mit einem zentralen Verbindungssitz (27) versehen ist, um eine selektiv lösbare Verbindung mit dem Befestigungskopf (29) zu ermöglichen, wobei der Befestigungskopf (29) umfasst:
einen vorstehenden ringförmigen Flansch (85), der an dem Befestigungsteil (24) anliegt,
einen Verbindungsabschnitt (29a), der von dem vorstehenden ringförmigen Flansch (85) herausragt
**dadurch gekennzeichnet, dass**
der zentrale Verbindungssitz (27) eine sechseckige Form aufweist und der Verbindungsabschnitt (29a) einen sechseckig geformten Bereich aufweist, die Klemmeinrichtung (84) ein magnetisches Element (89) umfasst, das konfiguriert ist,
um korrespondierend mit dem Verbindungssitz (27) selektiv mit dem Befestigungsteil (24) in magnetischen Kontakt zu treten, wobei der Befestigungskopf (29) einen Hals (88) aufweist, die an dem ringförmigen Flansch (85) ausgeformt ist und den Verbindungsabschnitt (29a) umschließt und wobei das magnetische Element (89) in dem Hals (88) angeordnet ist.

2. Fräswerkzeug nach Anspruch 1,
**dadurch gekennzeichnet, dass**
zumindest der Schneidteil (81, 82) aus Titan hergestellt ist.

3. Fräswerkzeug nach Anspruch 1 oder 2,
**dadurch gekennzeichnet, dass**
der Trägerteil (21, 32) aus Titan hergestellt ist.

4. Fräswerkzeug nach Anspruch 1, 2 oder 3,
**dadurch gekennzeichnet, dass**
der Befestigungsteil (24) aus Titan hergestellt ist.

5. Fräswerkzeug nach einem der vorhergehenden Ansprüche,
**dadurch gekennzeichnet, dass**
es vollständig aus Titan hergestellt ist.

6. Fräswerkzeug nach einem der vorhergehenden Ansprüche,
**dadurch gekennzeichnet, dass**
der Schneidteil (81, 82) und der Trägerteil (21, 32) zu einem einzigen Körper miteinander verbunden sind.

7. Fräswerkzeug nach einem der vorhergehenden Ansprüche,
**dadurch gekennzeichnet, dass**
der Trägerteil (21) eine Dicke (S2) zwischen 0,8 mm und 1,2 mm aufweist.

8. Fräswerkzeug nach einem der vorhergehenden Ansprüche,
**dadurch gekennzeichnet, dass**
der Befestigungsteil (24) eine Dicke (Si) zwischen 0,9 mm und 1,1 mm aufweist.

9. Fräswerkzeug nach einem der vorhergehenden Ansprüche,
**dadurch gekennzeichnet, dass**
der Befestigungskopf (29) einen sechseckigen Querschnitt aufweist, der mit der Form des Verbindungssitzes (27) zusammenpasst.

10. Fräswerkzeug nach einem der vorhergehenden Ansprüche,
**dadurch gekennzeichnet, dass**
der Befestigungsteil (24) ein Umfangsband (25) von kontinuierlicher Ringform und drei zentrale Speichen (37) umfasst, die im Wesentlichen im gleichen Winkel beabstandet sind.

11. Fräswerkzeug nach einem der vorhergehenden Ansprüche 1 bis 9,
**dadurch gekennzeichnet, dass**
der Befestigungsteil (24) von einem Stab gebildet ist, der mit dem zentralen Verbindungssitz (27) versehen ist und nur zwei diametral gegenüberliegende Speichen (37) definiert.

12. Fräswerkzeug nach einem der vorangehenden Ansprüche,
**dadurch gekennzeichnet, dass**
der Befestigungsteil (24) eine ringförmige Umfangsstufenkante (86) aufweist, die mit einem ringförmigen Umfangsrand (26) des Trägerteils (21) zusammenwirkt und daran anliegt, wobei das Befestigungsteil (24) mit dem Trägerteil (21) verschweißt ist, wobei die Umfangsstufenkante (86) einen ringförmigen Abschnitt (86a), der den Abschnitt des Befestigungsteils (24) verengt, der in das Innere des Trägerteils (21) zurückgesetzt ist, und einen ringförmigen Anschlagabschnitt (86b) definiert, der kopfwärts mit einer ringförmigen äußeren Umfangskante (26) des Trägerteils (21) verbunden ist.

13. Fräswerkzeug nach einem der vorhergehenden Ansprüche 1 bis 11,
**dadurch gekennzeichnet, dass**
der Befestigungsteil (24) einen glatten Umfangsrand (25a) aufweist, der mit dem Trägerteil (21) verschweißt und vollständig mit einem ringförmigen äußeren Umfangsrand (26) des Trägerteils (21) bündig eingelassen ist.

14. Fräswerkzeug nach einem der vorhergehenden Ansprüche,
**dadurch gekennzeichnet, dass**
das magnetische Element (89) eine ringförmige Form hat, die im Wesentlichen mit der Form des Hals (88) übereinstimmt.

15. Fräswerkzeug nach Anspruch 14,
**dadurch gekennzeichnet, dass**
das magnetische Element (89) so konfiguriert ist, dass es eine gleichmäßige Verbindungskraft auf den Befestigungsteil (24) überträgt, wobei die Ringform des magnetischen Elements (89) es ermöglicht, auf den gesamten Umfangsbereich um den Verbindungssitz (27) zu wirken.

## Revendications

1. Outil de fraisage pour opérations de chirurgie prothétique, comprenant :
une pièce de support (21, 32) de forme sensiblement hémisphérique et faite en métal, pourvue d'une pièce de coupe (81, 82) ayant une pluralité d'éléments coupants saillants (22) ;
une pièce de fixation (24) faite en métal et reliée de façon permanente à ladite pièce de support (21, 32) par soudage (31),
une tête de fixation (29) munie de moyens de serrage (84) aptes à être sélectivement activés pour une liaison stable et libérable à ladite pièce de fixation (24), ladite tête de fixation (29) étant apte à être reliée à un dispositif de manipulation (28),
ladite pièce de fixation (24) étant pourvue d'un siège central de liaison (27), afin de permettre une liaison sélectivement libérable avec ladite tête de fixation (29),
la tête de fixation (29) comprenant :
un bourrelet annulaire saillant (85) qui vient en butée contre la pièce de fixation (24),
une portion de liaison (29a) faisant saillie du bourrelet annulaire saillant (85),
**caractérisé en ce que**
le siège central de liaison (27) a une forme hexagonale et la portion de liaison (29a) a une section de forme hexagonale,
le moyen de serrage (84) comprend un élément magnétique (89) configuré pour entrer sélectivement en contact magnétique avec la pièce de fixation (24), en correspondance avec le siège de liaison (27),
la tête de fixation (29) comprenant
une gorge (88), aménagée sur le bourrelet annulaire saillant (85) et circonscrit à la portion de liaison (29a),
et l'élément magnétique (89) étant positionné dans la gorge (88).

2. Outil de fraisage selon la revendication 1, **caractérisé en ce qu'**au moins ladite pièce de coupe (81, 82) est faite en titane.

3. Outil de fraisage selon la revendication 1 ou la revendication 2, **caractérisé en ce que** la pièce de support (21, 32) est en titane.

4. Outil de fraisage selon la revendication 1, 2 ou 3, **caractérisé en ce que** la partie de fixation (24) est faite de titane.

5. Outil de fraisage selon l'une quelconque des revendications précédentes, **caractérisé en ce qu'**il est entièrement fait en titane.

6. Outil de fraisage selon l'une quelconque des revendications précédentes, **caractérisé en ce que** ladite pièce de coupe (81, 82) et ladite pièce de support (21, 32) sont réalisées en un seul corps l'une avec l'autre.

7. Outil de fraisage selon l'une quelconque des revendications précédentes, **caractérisé en ce que** ladite pièce de support (21) a une épaisseur (S2) comprise entre 0,8 mm et 1,2 mm.

8. Outil de fraisage selon l'une quelconque des revendications précédentes, **caractérisé en ce que** ladite pièce de fixation (24) a une épaisseur (S 1) comprise entre 0,9 mm et 1,1 mm.

9. Outil de fraisage selon l'une quelconque des revendications précédentes, **caractérisé en ce que** ladite tête de fixation (29) a une forme de section hexagonale, correspondant à la forme dudit siège de liaison (27).

10. Outil de fraisage selon l'une quelconque des revendications précédentes, **caractérisé en ce que** ladite pièce de fixation (24) comprend une bande périphérique (25) de forme annulaire continue et trois rayons centraux (37) sensiblement équidistants angulairement.

11. Outil de fraisage selon l'une quelconque des revendications 1 à 9 précédentes, **caractérisé en ce que** ladite pièce de fixation (24) est formée par une barre munie dudit siège central de liaison (27) et définissant seulement deux rayons (37) diamétralement opposés.

12. Outil de fraisage selon l'une quelconque des revendications précédentes, **caractérisé en ce que** ladite pièce de fixation (24) présente un bord périmétrique en gradins (86) de forme annulaire qui se relie à un bord périphérique annulaire (26) de ladite pièce de support (21) et vient en butée avec celui-ci, ladite pièce de fixation (24) étant soudée à ladite pièce de support (21), le bord périmétrique en gradins (86) définissant une partie annulaire (86a) qui rétrécit la section de ladite pièce de fixation (24) en retrait à l'intérieur de ladite pièce de support (21), et une partie annulaire de butée (86b) reliée dans le sens de la tête à un bord externe périphérique annulaire (26) de ladite pièce de support (21).

13. Outil de fraisage selon l'une quelconque des revendications 1 à 11 précédentes, **caractérisé en ce que** ladite pièce de fixation (24) présente un bord périphérique lisse (25a) qui se relie à ladite pièce de support (21) par soudage et étant complètement en retrait, de façon affleurante à un bord externe périphérique annulaire (26) de ladite pièce de support (21).

14. Outil de fraisage selon l'une quelconque des revendications précédentes, **caractérisé en ce que** ledit élément magnétique (89) a une forme annulaire correspondant sensiblement à la forme de la gorge (88).

15. Outil de fraisage selon la revendication 14, **caractérisé en ce que** l'élément magnétique (89) est configuré pour transmettre une force de liaison uniforme sur la pièce de fixation (24), la forme annulaire de l'élément magnétique (89) permettant d'agir sur l'ensemble de la zone périmétrique autour du siège de liaison (27).
